# EUROPEAN PATENT APPLICATION

(11) **EP 2 559 761 A1**
(43) Date of publication of application: **20.02.2013**
(21) Application number: 11768615.4
(22) Date of filing: 11.04.2011
(51) Int. Cl.: C12N 15/09, C12Q 1/68

(54) **PRIMER SET FOR PCR, REACTION LIQUID FOR PCR, AND METHOD FOR DETECTING FOOD POISONING BACTERIA**

(30) Priority: 14.04.2010 JP 2010093316
(71) Applicant: Toyo Seikan Kaisha, Ltd., Shinagawa-ku Tokyo 141-8640 (JP); Toyo Kohan Co., Ltd., Tokyo 102-8447 (JP)
(72) Inventor: YAMASAKI, Takaaki, Yokohama-shi Kanagawa 240-0062 (JP); HARADA, Takaaki, Yokohama-shi Kanagawa 230-0001 (JP); SARUWATARI, Yoshihiro, Yokohama-shi Kanagawa 240-0062 (JP); KAMEI, Shuichi, Kudamatsu-shi Yamaguchi 744-8611 (JP)
(74) Representative: Liebetanz, Michael
(86) International application number: PCT/JP2011/002126
(87) International publication number: WO 2011/129091

(57) **Abstract**

Nucleic acid of two or more types of food poisoning bacteria among *Bacillus cereus, Campylobacter, Escherichia coli, Listeria, Salmonella, Staphylococcus aureus,* and *Vibrio parahaemolyticus* is specifically amplified to simultaneously and specifically detect the food poisoning bacteria. More specifically, nucleic acid of food poisoning bacteria is amplified by PCR using a PCR primer set that includes two or more primer sets selected from a primer set for amplifying DNA of *Bacillus cereus* (SEQ ID NO: 1 and SEQ ID NO: 2), a primer set for amplifying DNA of *Bacillus cereus* (SEQ ID NO: 3 and SEQ ID NO: 4), a primer set for amplifying DNA of *Campylobacter* (SEQ ID NO: 5 and SEQ ID NO: 6), a primer set for amplifying DNA of *Escherichia coli* (SEQ ID NO: 7 and SEQ ID NO: 8), a primer set for amplifying DNA of *Listeria* (SEQ ID NO: 9 and SEQ ID NO: 10), a primer set for amplifying DNA of *Salmonella* (SEQ ID NO: 11 and SEQ ID NO: 12), a primer set for amplifying DNA of *Staphylococcus aureus* (SEQ ID NO: 13 and SEQ ID NO: 14), and a primer set for amplifying DNA of *Vibrio parahaemolyticus* (SEQ ID NO: 15 and SEQ ID NO: 16).

SEQUENCE LISTING

<110>  TOYO SEIKAN KAISHA,LTD
       Toyo Kohan Co., Ltd.

<120>  A primer set for PCR, a reaction liquid for PCR, and a method for
detecting food poisoning bacteria

<130>  SP2201A

<160>  16

<170>  PatentIn version 3.1

<210>  1
<211>  31
<212>  DNA
<213>  Bacillus cereus

<400>  1
catctgttga tgcggcttta aaagggaaag t                                    31


<210>  2
<211>  30
<212>  DNA
<213>  Bacillus cereus

<400>  2
gagtcgcttt atcctttgca tctaccgcag                                      30


<210>  3
<211>  30
<212>  DNA
<213>  Bacillus cereus

<400>  3
cacctgccgg aggagcaaaa atgatacaac                                      30


<210>  4
<211>  31
<212>  DNA
<213>  Bacillus cereus

<400>  4
cagattcatt cttcgcttat ggtggtgact c                                    31


<210>  5
<211>  26
<212>  DNA
<213>  Campylobacter jejuni

<400>  5
ggtgatatct acggatttta ccccta                                          26


<210>  6
<211>  27
<212>  DNA
<213>  Campylobacter jejuni

<400>  6

aatgggtatt cttggtgata tctacgg 27

<210> 7
<211> 31
<212> DNA
<213> Escherichia coli

<400> 7
cgctcgtctg atgtccgcta ttccattgaa t 31

<210> 8
<211> 26
<212> DNA
<213> Escherichia coli

<400> 8
accacgcagg caagctgctg agtcgg 26

<210> 9
<211> 27
<212> DNA
<213> Listeria monocytogenes

<400> 9
cttcaaatcc agagaatcct ccaccgc 27

<210> 10
<211> 26
<212> DNA
<213> Listeria monocytogenes

<400> 10
gagaatcctc caccgctaaa tccgcc 26

<210> 11
<211> 30
<212> DNA
<213> Salmonella enterica

<400> 11
gaacaaccca tttgtattgg ttgttacggc 30

<210> 12
<211> 23
<212> DNA
<213> Salmonella enterica

<400> 12
ggctgctcgc ctttgctggt ttt 23

<210> 13
<211> 24
<212> DNA
<213> Staphylococcus aureus

<400> 13
cacgcctgga gagccttcac cagc 24

<210>  14
<211>  30
<212>  DNA
<213>  Staphylococcus aureus

<400>  14
gttactgtaa tggcgctggt catgtagctg                                        30


<210>  15
<211>  41
<212>  DNA
<213>  Vibrio parahaemolyticus

<400>  15
tcagtttact tttttgggtt ttttggcttt catgaaacct g                           41


<210>  16
<211>  34
<212>  DNA
<213>  Vibrio parahaemolyticus

<400>  16
tcattaatgt tcacagtcat gtaggatgtc agcc                                   34

## Description

### TECHNICAL FIELD

The invention relates to a PCR primer set for detecting food poisoning bacteria. More specifically, the invention relates to a PCR primer set, a PCR reaction mixture, and a method for detecting food poisoning bacteria that make it possible to specifically detect a plurality of types of food poisoning bacteria.

### BACKGROUND ART

The presence or absence of food poisoning bacteria has been checked in the fields of food inspection, environmental inspection, clinical trial, animal health management, and the like. For example, deoxyribonucleic acid (DNA) contained in a sample is amplified by a polymerase chain reaction (PCR), and the presence or absence of the target bacteria is determined based on the amplified product.
In this case, it is important to design the primers so that the target bacteria can be specifically amplified.

Patent Document 1 discloses a primer set that makes it possible to detect enterohemorrhagic *Escherichia coli O157, Salmonella, Campylobacter, Listeria,* and *Staphylococcus aureus.*
Patent Document 2 discloses a primer set for amplifying DNA of *Salmonella* O4 using the loop-mediated isothermal amplification (LAMP) method.

Patent Document 1: JP-A-2007-274934
Patent Document 2: JP-A-2008-72951

### SUMMARY OF THE INVENTION

### TECHNICAL PROBLEM

However, other food poisoning bacteria cannot be detected using the above method. Specifically, bacterial food poisoning also frequently occurs due to *Bacillus cereus* and *Vibrio parahaemolyticus,* and 90% or more of diseases due to bacterial food poisoning are caused by *Escheriehia coli, Listeria, Salmonella, Campylobacter, Staphylococcus aureus, Bacillus cereus,* and *Vibrio parahaemolyticus.*
Therefore, it is very advantageous to make it possible to simultaneously and specifically detect whether these food poisoning bacteria are present in food, the environment, and the like.

In order to specifically detect different types of food poisoning bacteria, it is necessary to design the primers so that a region (part) of DNA of the different types of food poisoning bacteria can be simultaneously amplified. In this case, it is necessary to prevent a situation in which the non-target region is amplified due to a combination of primers of primer sets that differ in target region. Since a plurality of primer sets that differ in target region may compete when each primer has a similar length or melting temperature, it is necessary to design the primers so that amplification with higher specificity can be implemented as compared with the case of using a single primer set.
It has been very difficult to specifically detect a plurality of types of food poisoning bacteria, and it has been considered that only up to five types of food poisoning bacteria can be specifically detected.

In view of the above situation, the inventors of the invention conducted extensive studies and experiments, and developed a multiplex PCR primer set that can specifically amplify a toxin region gene or essential gene (eight genes) of *Escherichia coli, Listeria, Salmonella, Campylobacter, Staphylococcus aureus, Bacillus cereus,* and *Vibrio parahaemolyticus* to complete the invention.
Specifically, an object of the invention is to provide a PCR primer set, a PCR reaction mixture, a method for detecting food poisoning bacteria, and a primer design method that make it possible to specifically amplify two or more types of food poisoning bacteria among *Bacillus cereus, Campylobacter, Escherichia coli, Listeria, Salmonella, Staphylococcus aureus,* and *Vibrio parahaemolyticus.*

### SOLUTION TO PROBLEM

According to one aspect of the invention, a PCR primer set for amplifying nucleic acid by PCR includes two or more primer sets selected from a group consisting of a first primer set for amplifying DNA of *Bacillus cereus,* the first primer set comprising a primer having a base sequence of SEQ ID NO: 1 and a primer having a base sequence of SEQ ID NO: 2, a second primer set for amplifying DNA of *Bacillus cereus,* the second primer set comprising a primer having a base sequence of SEQ ID NO: 3 and a primer having a base sequence of SEQ ID NO: 4, a third primer set for amplifying DNA of *Campylobacter,* the third primer set comprising a primer having a base sequence of SEQ ID NO: 5 and a primer having a base sequence of SEQ ID NO: 6, a fourth primer set for amplifying DNA of *Escherichia coli,* the fourth primer set comprising a primer having a base sequence of SEQ ID NO: 7 and a primer having a base sequence of SEQ ID NO: 8, a fifth primer set for amplifying DNA of *Listeria,* the fifth primer set comprising a primer having a base sequence of SEQ ID NO: 9 and a primer having a base sequence of SEQ ID NO: 10, a sixth primer set for amplifying DNA of *Salmonella,* the sixth primer set comprising a primer having a base sequence of SEQ ID NO: 11 and a primer having a base sequence of SEQ ID NO: 12, a seventh primer set for amplifying DNA of *Staphylococcus aureus,* the seventh primer set comprising a primer having a base sequence of SEQ ID NO: 13 and a primer having a base sequence of SEQ ID NO: 14, and an eighth primer set for amplifying DNA of *Vibrio parahaemolyticus,* the eighth primer set comprising a primer having a base sequence of SEQ ID NO: 15 and a primer having a base sequence of SEQ ID NO: 16.

According to another aspect of the invention, a PCR primer set for amplifying nucleic acid by PCR includes two or more primer sets selected from a group consisting of a primer set for amplifying a non-hemolytic enterotoxin (nhe) gene contained in genomic DNA of *Bacillus cereus,* a primer set for amplifying a cereulide synthetase (cesB) gene contained in genomic DNA of *Bacillus cereus,* a primer set for amplifying a ribosomal (16S rDNA) gene contained in genomic DNA of *Campylobacter,* a primer set for amplifying a uridine monophosphate kinase (pyrH) gene contained in genomic DNA of *Escherichia coli,* a primer set for amplifying a heat shock protein (dnaJ) gene contained in genomic DNA of *Listeria,* a primer set for amplifying an invasion (invA) gene contained in genomic DNA of *Salmonella,* a primer set for amplifying a heat shock protein (dnaJ) gene contained in genomic DNA of *Staphylococcus aureus,* and a primer set for amplifying a thermostable direct hemolysin (tdh) gene contained in genomic DNA of *Vibrio parahaemolyticus.*

According to another aspect of the invention, a PCR reaction mixture includes the PCR primer set.

According to another aspect of the invention, a method for detecting food poisoning bacteria includes specifically amplifying nucleic acid contained in a PCR amplification target sample using two or more primer sets to obtain an amplified product, when the PCR amplification target sample contains nucleic acid of one or two or more types of food poisoning bacteria that correspond to the two or more primer sets, and detecting the one or two or more types of food poisoning bacteria based on the amplified product, the two or more primer sets being selected from a group consisting of a first primer set for amplifying DNA of *Bacillus cereus,* the first primer set comprising a primer having a base sequence of SEQ ID NO: 1 and a primer having a base sequence of SEQ ID NO: 2, a second primer set for amplifying DNA of *Bacillus cereus,* the second primer set comprising a primer having a base sequence of SEQ ID NO: 3 and a primer having a base sequence of SEQ ID NO: 4, a third primer set for amplifying DNA of *Campylobacter,* the third primer set comprising a primer having a base sequence of SEQ ID NO: 5 and a primer having a base sequence of SEQ ID NO: 6, a fourth primer set for amplifying DNA of *Escherichia coli,* the fourth primer set comprising a primer having a base sequence of SEQ ID NO: 7 and a primer having a base sequence of SEQ ID NO: 8, a fifth primer set for amplifying DNA of *Listeria,* the fifth primer set comprising a primer having a base sequence of SEQ ID NO: 9 and a primer having a base sequence of SEQ ID NO: 10, a sixth primer set for amplifying DNA of *Salmonella,* the sixth primer set comprising a primer having a base sequence of SEQ ID NO: 11 and a primer having a base sequence of SEQ ID NO: 12, a seventh primer set for amplifying DNA of *Staphylococcus aureus,* the seventh primer set comprising a primer having a base sequence of SEQ ID NO: 13 and a primer having a base sequence of SEQ ID NO: 14, and an eighth primer set for amplifying DNA of *Vibrio parahaemolyticus,* the eighth primer set comprising a primer having a base sequence of SEQ ID NO: 15 and a primer having a base sequence of SEQ ID NO: 16.

### ADVANTAGEOUS EFFECTS OF THE INVENTION

The invention thus makes it possible to specifically amplify two or more types of food poisoning bacteria among *Bacillus cereus, Campylobacter, Escherichia coli, Listeria, Salmonella, Staphylococcus aureus,* and *Vibrio parahaemolyticus.* This makes it possible to specifically detect the food poisoning bacteria.

### BRIEF DESCRIPTION OF DRAWINGS

FIG. 1 is a view showing the SEQ ID NO., the F/R classification, the target food poisoning bacteria, the amplification target region, and the length of the amplified product corresponding to the novel primer set according to the embodiment of the invention.
FIG. 2 is a view showing the relationship between the amplification target region and the sequence of the novel primer set according to the embodiment of the invention.
FIG. 3 is a view showing the amplification results for a non-hemolytic enterotoxin gene contained in genomic DNA of *Bacillus cereus (Bacillus cereus*/*nhe)* (Experiment 1).
FIG. 4 is a view showing the amplification results for a cereulide synthetase gene contained in genomic DNA of *Bacillus cereus (Bacillus* cereus/cesB) (Experiment 2).
FIG. 5 is a view showing the amplification results for a ribosomal gene contained in genomic DNA of *Campylobacter (Campylobacter*/*16S* rDNA) (Experiment 3).
FIG. 6 is a view showing the amplification results for a uridine monophosphate kinase gene contained in genomic DNA of *Escherichia coli (Escherichia* coli/pyrH) (Experiment 4).
FIG. 7 is a view showing the amplification results for a heat shock protein gene contained in genomic DNA of *Listeria (Listeria monocytogenes*/*dnaJ)* (Experiment 5).
FIG. 8 is a view showing the amplification results for an invasion gene contained in genomic DNA of *Salmonella (Salmonella enterica*/*invA)* (Experiment 6).
FIG. 9 is a view showing the amplification results for a heat shock protein gene contained in genomic DNA of *Staphylococcus aureus (Staphylococcus aureus*/*dnaJ)* (Experiment 7).
FIG. 10 is a view showing the amplification results for a thermostable direct hemolysin gene contained in genomic DNA of *Vibrio parahaemolyticus (Vibrio parahaemolyticus*/*tdh)* (Experiment 8).
FIG. 11 is a view showing the results when separately amplifying a region (part) of genomic DNA of seven types of food poisoning bacteria using the primers of eight primer sets (Example 1).
FIG. 12 is a view showing the results when simultaneously amplifying a region (part) of genomic DNA of seven types of food poisoning bacteria using the primers of eight primer sets (Example 2).

### DESCRIPTION OF EMBODIMENTS

Embodiments of the invention are described in detail below.

### PCR primer set

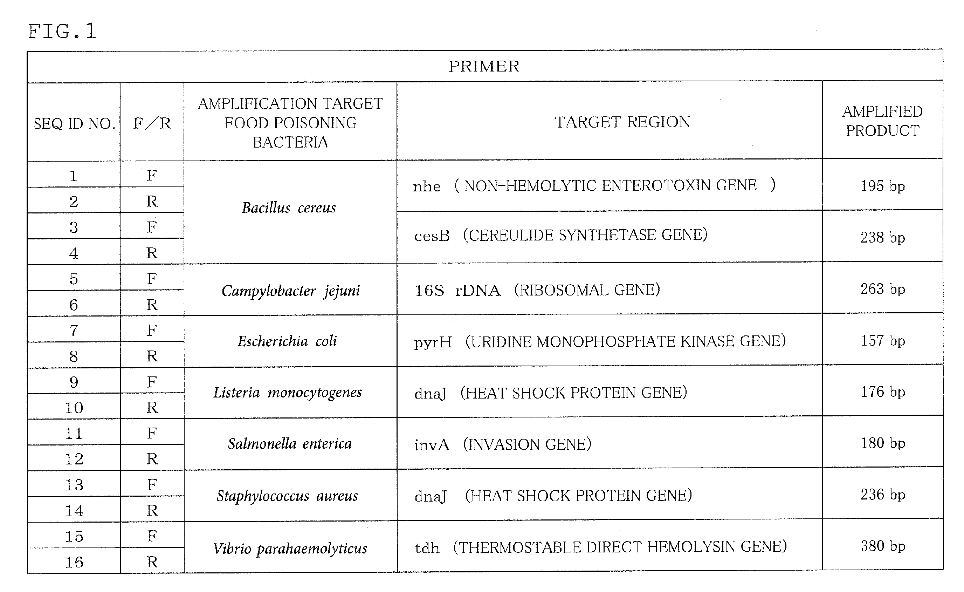

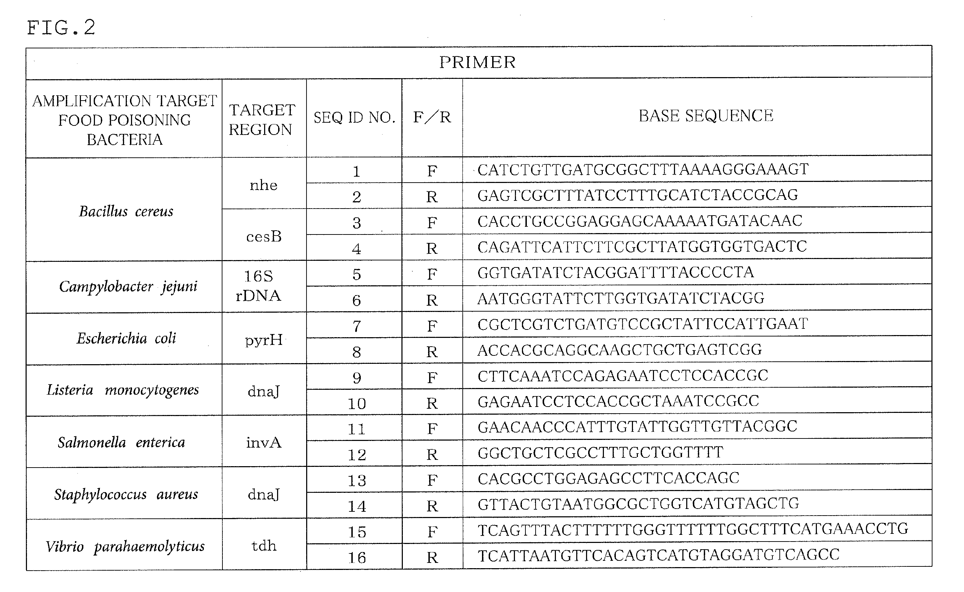

A PCR primer set according to an embodiment of the invention is described below with reference to FIGS. 1 and 2.
The PCR primer set is used to amplify a region (part) of genomic DNA in a sample (hereinafter may be referred to as "genomic DNA sample" or "DNA sample") by PCR. More specifically, the PCR primer set is used to amplify a region specified by a forward primer and a reverse primer. The PCR primer set is added to a PCR reaction mixture. The PCR reaction mixture includes a nucleic acid substrate, a nucleic acid polymerase, a genomic DNA sample, a buffer solution, and the like in addition to the PCR primer set. A region (part) of the genomic DNA sample is amplified using the PCR reaction mixture utilizing a nucleic acid amplification system (e.g., thermal cycler). More specifically, when the sample contains genomic DNA having a target region (amplification target region) that is amplified using the PCR primer set, the target region is amplified. The PCR primer set according to the embodiment of the invention may be used for normal PCR.

In FIG. 1, the item "SEQ ID NO." indicates the SEQ ID NO. of each base sequence listed in the Sequence Listing. The item "F/R" indicates whether the primer is a forward primer or a reverse primer (F: forward primer, R: reverse primer). The item "amplification target food poisoning bacteria" indicates the scientific name of food poisoning bacteria that are amplified using the PCR primer set according to the embodiment of the invention. The item "target region" indicates the name of a gene present in the target region of the food poisoning bacteria that is amplified using the PCR primer set according to the embodiment of the invention. The item "amplified product" indicates the length of the base sequence of an amplified product obtained by amplifying the corresponding target region using the PCR primer set according to the embodiment of the invention. FIG. 2 shows the base sequences of each PCR primer set corresponding to the amplification target region of the food poisoning bacteria.

SEQ ID NO: 1 and SEQ ID NO: 2 indicate the base sequences of the primer set for amplifying a non-hemolytic enterotoxin (nhe) gene contained in genomic DNA of *Bacillus cereus.* The length of the amplified product is 195 base pairs (bp).
SEQ ID NO: 3 and SEQ ID NO: 4 indicate the base sequences of the primer set for amplifying a cereulide synthetase (cesB) gene contained in genomic DNA of *Bacillus cereus.* The length of the amplified product is 238 bp.
SEQ ID NO: 5 and SEQ ID NO: 6 indicate the base sequences of the primer set for amplifying a ribosomal (16S rDNA) gene contained in genomic DNA of *Campylobacter.* The length of the amplified product is 263 bp.
SEQ ID NO: 7 and SEQ ID NO: 8 indicate the base sequences of the primer set for amplifying a uridine monophosphate kinase (pyrH) gene contained in genomic DNA of *Escherichia coli.* The length of the amplified product is 157 bp.

SEQ ID NO: 9 and SEQ ID NO: 10 indicate the base sequences of the primer set for amplifying a heat shock protein (dnaJ) gene contained in genomic DNA of *Listeria.* The length of the amplified product is 176 bp.
SEQ ID NO: 11 and SEQ ID NO: 12 indicate the base sequences of the primer set for amplifying an invasion (invA) gene contained in genomic DNA of *Salmonella.* The length of the amplified product is 180 bp.
SEQ ID NO: 13 and SEQ ID NO: 14 indicate the base sequences of the primer set for amplifying a heat shock protein (dnaJ) gene contained in genomic DNA of *Staphylococcus aureus.* The length of the amplified product is 236 bp.
SEQ ID NO: 15 and SEQ ID NO: 16 indicate the base sequences of the primer set for amplifying a thermostable direct hemolysin (tdh) gene contained in genomic DNA of *Vibrio parahaemolyticus.* The length of the amplified product is 380 bp.
Each base sequence is written in the 5' to 3' direction.

Each primer of the PCR primer set according to the embodiment of the invention is not limited to the above base sequence. Each primer may be a primer having the corresponding base sequence wherein one or a plurality of bases are deleted, substituted, or added. Each primer may be a primer that includes a nucleic acid fragment that hybridizes to a nucleic acid fragment having a base sequence complementary to the corresponding base sequence under stringent conditions.

The term "stringent conditions" used herein refers to conditions under which a specific hybrid is formed, but a non-specific hybrid is not formed. For example, the stringent conditions may be conditions under which DNA having high homology (90% or more, and preferably 95% or more) with DNA having a sequence among the sequences of SEQ ID NO: 1 to 16 hybridizes to DNA having a base sequence complementary to that of DNA having the sequence among the sequences of SEQ ID NO: 1 to 16. The stringent conditions normally refer to conditions under which hybridization occurs at a temperature lower than the melting temperature (Tm) of a perfect hybrid by about 5°C to about 30°C (preferably about 10°C to about 25°C). For example, the conditions described in J. Sambrook et al., Molecular Cloning, A Laboratory Manual, Second Edition, Cold Spring Harbor Laboratory Press (1989) (particularly the conditions described in §11.45 "Conditions for Hybridization of Oligonucleotide Probes") may be used as the stringent conditions.

It is also possible to use a PCR primer set that includes primers respectively having a base sequence complementary to the PCR primer set according to the embodiment of the invention.
More specifically, a PCR primer set that includes primers respectively having a base sequence (in the 3' to 5' direction) complementary to those of the primers included in the PCR primer set according to the embodiment of the invention, may be used.

When the sample included in the PCR reaction mixture contains genomic DNA of one type of the amplification target food poisoning bacteria, and a PCR amplification reaction is effected using the PCR reaction mixture that includes the above eight PCR primer sets, the amplification target region can be specifically amplified.
When the sample contains genomic DNA of two or more types of the amplification target food poisoning bacteria, the amplification target region of each genomic DNA can be simultaneously and specifically amplified. When the sample contains genomic DNA (i.e., eight genes) of all types (seven types) of the amplification target food poisoning bacteria, the amplification target region of each genomic DNA can be simultaneously and specifically amplified.

Specifically, when using the PCR primer set according to the embodiment of the invention, competition does not occur between genomic DNA of the target food poisoning bacteria and genomic DNA other than genomic DNA of the target food poisoning bacteria. Moreover, non-specific amplification due to a combination of primers included in different primer sets does not occur even when using a plurality of PCR primer sets in combination.
Therefore, when the sample contains genomic DNA of food poisoning bacteria having the amplification target region, the amplification target region can be specifically amplified by adding the PCR primer set according to the embodiment of the invention to the PCR reaction mixture.
Moreover, the amplification target region of each genomic DNA can be simultaneously and specifically amplified even when the sample contains a plurality of types of the amplification target food poisoning bacteria.

When the PCR reaction mixture includes one to seven PCR primer sets among the eight PCR primer sets, the target region can also be specifically amplified when the PCR reaction mixture contains genomic DNA of food poisoning bacteria having the amplification target region.

### PCR reaction mixture

A PCR reaction mixture according to an embodiment of the invention includes at least one PCR primer set among the eight PCR primer sets. The PCR reaction mixture preferably includes two or more PCR primer sets among the eight PCR primer sets, and more preferably includes all of the eight PCR primer sets. The PCR reaction mixture may include commonly-used components in addition to the PCR primer set(s).
For example, the PCR reaction mixture may have the following composition. Note that the composition of the PCR reaction mixture is not limited thereto. In particular, the volume of the primers, the DNA sample, and sterilized water varies depending on the number of types of amplification target food poisoning bacteria.

Buffer solution (10 vol%)
Nucleic acid substrate (8 vol%)
Forward primer (10 ng/µl, 2 to 16 vol%)
Reverse primer (10 ng/µl, 2 to 16 vol%)
Nucleic acid polymerase (0.5 vol%)
DNA sample (5 to 35 vol%)
Water (14.5 to 72.5 vol%)

### Method for detecting food poisoning bacteria

A method for detecting food poisoning bacteria according to an embodiment of the invention is described below.
A genome contained in a sample is amplified by PCR using the PCR reaction mixture according to the embodiment of the invention.
When detecting food poisoning bacteria in practice, a sample is collected from food, the environment, or the like, and bacteria contained in the sample are cultured. Genomic DNA is extracted from the cultured bacteria, and used as the sample included in the PCR reaction mixture. Therefore, it is unknown whether or not the amplification target food poisoning bacteria are present in the sample. The amplification target food poisoning bacteria are detected when the amplification target food poisoning bacteria are present in the sample. When the PCR reaction mixture includes all of the eight PCR primer sets, and at least one gene among the eight genes (regions) of the seven types of bacteria is present in the sample, the amplification target region can be specifically amplified.

A nucleic acid amplification system is used when amplifying a gene by PCR. A normal thermal cycler or the like may be used as the nucleic acid amplification system. PCR may be performed under the following conditions, for example. Note that the PCR reaction conditions are not limited thereto.
(1) 95°C/2min
(2) 95°C/10 sec (DNA strand separation step (denaturation step))
(3) 68°C/30 sec (annealing step)
(4) 72°C/30 sec (DNA synthesis step)
(5) 72°C/2 min
The steps (2) to (4) are repeated in 40 cycles.

The amplified product is subjected to electrophoresis to check whether or not the amplified product has been obtained due to the PCR primer set according to the embodiment of the invention.
Electrophoresis may be implemented by agarose gel electrophoresis, acrylamide gel electrophoresis, microchip electrophoresis, or the like.

### Primer design method

A primer design method according to an embodiment of the invention is described below.
In order to simultaneously and specifically detect different types of food poisoning bacteria, it is necessary to design the primers so that DNA of the different types of food poisoning bacteria can be simultaneously amplified (see the section entitled "BACKGROUND ART"). However, a plurality of primer sets that differ in target region may compete when each primer has a similar length or melting temperature. Therefore, it is necessary to design the primers so that amplification with higher specificity can be implemented as compared with the case of using a single primer set.
The inventors conducted studies in order to implement a primer design method that can improve the specificity of each primer, and found that the specificity of each primer can be improved by satisfying the following requirements.

### (1) Melting temperature

A primer is designed to have a melting temperature of 70°C or more.
A primer having a melting temperature of 55 to 60°C has been normally used. It is conjectured that the PCR primer set according to the embodiment of the invention increases the free energy during a PCR amplification reaction to suppress a non-specific amplification reaction due to the primers having a high melting temperature.

### (2) GC content

A primer is designed to have a base sequence having a GC content of 60% or more.
A primer having a GC content of 40 to 60% has been normally used. It is conjectured that the PCR primer set according to the embodiment of the invention improves specific binding during the annealing step due to the primers having a high content of G and C that have a high binding capability as compared with A and T.

### (3) Length of primer

A primer is designed to have a base sequence length of about 23 mer to about 41 mer. The base sequence length of each primer is preferably about 26 mer to about 34 mer, and more preferably about 28 mer to about 32 mer.
A primer having a base sequence length of about 18 mer to about 25 mer has been normally used. The PCR primer set according to the embodiment of the invention eliminates non-specific amplification by utilizing a primer having a greater base sequence length.
It is possible to improve the specificity (i.e., a capability to specifically amplify the amplification target gene region) as compared with a known primer by designing the primers so that at least one of the requirements (1) to (3) is satisfied.
The PCR primer set according to the embodiment of the invention is designed to satisfy all of the requirements (1) to (3) so that it is very advantageous to specifically amplify the eight regions of the seven types of bacteria.

### (4) Detection target region

The PCR primer set according to the embodiment of the invention is designed so that the PCR primer set aims at the specific region of the respective food poisoning bacteria as described above with reference to FIG. 1.
Specifically, the PCR primer set according to the embodiment of the invention aims at the gene in the toxin region and/or the essential region of the respective food poisoning bacteria.

More specifically, a non-hemolytic enterotoxin (nhe) gene and a cereulide synthetase (cesB) gene are selected as the amplification target region of *Baciltus cereus,* a ribosomal (16S rDNA) gene is selected as the amplification target region of *Campylobacter,* a uridine monophosphate kinase (pyrH) gene is selected as the amplification target region of *Escherichia coli,* a heat shock protein (dnaJ) gene is selected as the amplification target region of *Listeria,* an invasion (invA) gene is selected as the amplification target region of *Salmonella,* a heat shock protein (dnaJ) gene is selected as the amplification target region of *Staphylococcus aureus,* and a thermostable direct hemolysin (tdh) gene is selected as the amplification target region of *Vibrio parahaemolyticus.*
The specificity of each primer of the PCR primer set according to the embodiment of the invention can be further improved by selecting these regions as the amplification target region.

The PCR primer set, the PCR reaction mixture, and the method for detecting food poisoning bacteria according to the embodiments of the invention thus make it possible to simultaneously and specifically amplify the eight regions of the seven food poisoning bacteria (i.e., *Bacillus cereus, Campylobacter, Escherichia coli, Listeria, Salmonella, Staphylococcus aureus,* and *Vibrio parahaemolyticus).* This makes it possible to simultaneously and specifically detect these food poisoning bacteria.
It is possible to design primers with high specificity (e.g., the PCR primer set according to the embodiment of the invention) by utilizing the primer design method according to the embodiment of the invention.

### EXAMPLES

A verification experiment was performed in order to check whether or not the amplification target region can be correctly amplified using the PCR primer set according to the embodiment of the invention.
Specifically, a PCR amplification reaction was effected by the above method using a PCR reaction mixture containing the PCR primer set according to the embodiment of the invention, and the amplified product was subjected to electrophoresis to check whether or not a correct amplified product was obtained. The verification experiment was performed using each of the eight PCR primer sets (see Experiments 1 to 8).

### Experiment 1

A PCR primer set including a primer having the base sequence of SEQ ID NO: 1 and a primer having the base sequence of SEQ ID NO: 2 was used. The amplification target region is a non-hemolytic enterotoxin (nhe) gene in the toxin region of genomic DNA of *Bacillus cereus.* The length of the amplified product is 195 bp.
The PCR reaction mixture had the following composition. The primers were synthesized by Life Technologies Japan Ltd. The other components are products manufactured by Takara Bio Inc.
Buffer solution "10×Ex Taq buffer" (20 mM Mg 2+ plus): 2.0 µl
Nucleic acid substrate "dNTP Mixture" (2.5 mM dATP, 2.5 mM dCTP, 2.5 mM dGTP, and 2.5 mM dTTP): 1.6 µl
primerF (10 ng/µl, final conc.: 4 ng): 0.4 µl
primerR (10 ng/µl, final conc.: 4 ng): 0.4 µl
Nucleic acid polymerase "EX Taq" (5 U/µl): 0.1 µl
DNA sample: 0.1 µl
Sterilized water: 14.5 µl
(total amount: 20 µl)

Genomic DNA of the following food poisoning bacteria strains 1 to 11 was used as the DNA sample, and separately subjected to PCR.
1. *Bacillus cereus* NBRC 15305
*2. Bacillus cereus* TIFT 114011
*3. Campylobacter coli* ATCC 43478
*4. Campylobacter jejuni* ATCC 33560
*5. Escherichia coli* NBRC 102203
*6. Listeria monocytogenes ATCC* 15313
*7. Salmonella enterica* ATCC 9270
*8. Staphylococcus aureus* NBRC 100910
*9. Vibrio parahaemolyticus* GTC 2055
10. *Vibrio parahaemolyticus* ATCC 17802
11. *Yersinia enterocolitica* ATCC 9610
12. Negative Cont.

These food poisoning bacteria strains were transferred from the following organizations.
Biological Resource Center (NBRC), National Institute of Technology and Evaluation Toyo Institute of Food Technology (TIFT)
American Type Culture Collection (ATCC)
Gifu University School of Medicine, Pathogenic Microorganism Genetic Resource Stock Center (GTC Collection)

Note that the experiment using the PCR reaction mixture containing *Yersinia enterocolitica* ATCC 9610 (strain 11) was performed to check whether or not each PCR primer set according to the embodiment of the invention shows a false-positive reaction. The PCR reaction mixture in which the DNA sample was replaced with an equal amount of sterilized water was used as the negative control.

PCR gene amplification was performed using a system "Mastercycler ep gradient" (manufactured by Eppendorf). PCR was performed under the following reaction conditions (i.e., the same reaction conditions as those described above).
(1) 95°C/2 min
(2) 95°C/10 sec (DNA strand separation step (denaturation step))
(3) 68°C/30 sec (annealing step)
(4) 72°C/30 sec (DNA synthesis step)
(5) 72°C/2 min
The steps (2) to (4) were repeated in 40 cycles.

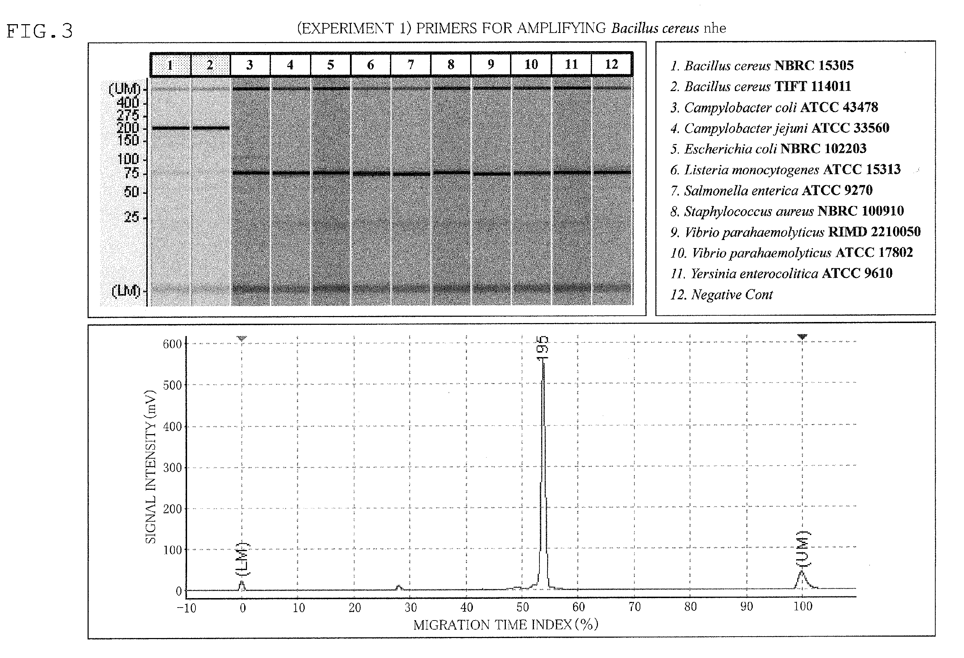

The PCR product was subjected to agarose gel electrophoresis corresponding to each amplification target region to check whether or not a correct amplified product was obtained. Electrophoresis was performed using an electrophoresis system "MultiNA" (manufactured by Shimadzu Corporation). The results are shown in FIG. 3.

The bands (lanes 1 to 12) shown in FIG. 3 indicate the electrophoresis results when subjecting each PCR product obtained using the PCR reaction mixture containing the corresponding DNA sample or the like to electrophoresis. The graph shown in FIG. 3 indicates the length of the amplified product. The graph indicates the peak of the amplified product regarding the band in which the target amplified product (i.e., the amplification target of the PCR primer set) was obtained. The above also applies to Experiments 2 to 8.

When subjecting the PCR amplified product to electrophoresis, the base sequence of the PCR amplified product does not completely coincide with the theoretical base sequence (i.e., approximate results are obtained) due to the effects of a reagent used for the electrophoresis system "MultiNA" (manufactured by Shimadzu Corporation) and the like.

The PCR primer set including a primer having the base sequence of SEQ ID NO: 1 and a primer having the base sequence of SEQ ID NO: 2 was used in Experiment 1. The amplification target region is a non-hemolytic enterotoxin (nhe) gene of *Bacillus cereus,* and the length of the amplified product is 195 bp.
As shown in FIG. 3, a band was observed at around 200 bp when using genomic DNA of *Bacillus cereus* (strains 1 and 2) as the DNA sample, and no band was observed at around 200 bp when using the strains 3 to 12. The graph shown in FIG. 3 indicates the results obtained using the strain 1. As shown in FIG. 3, a peak was observed at 195 bp.
It is considered that other bands (e.g., a band at around 25 bp and a band at 75 bp) in the electrophoresis results are attributed to the primer or a dimer formed by the primer. This also applies to Experiments 2 to 8.

The above results suggest that a non-hemolytic enterotoxin (nhe) gene of *Bacillus cereus* was correctly amplified using the PCR primer set used in Experiment 1 when using the strains 1 and 2 (genomic DNA of *Bacillus cereus)* as the DNA sample.
When using the strains 3 to 11 (genomic DNA other than genomic DNA of *Bacillus cereus)* as the DNA sample, a band that is considered to be attributed to a dimer formed by the primer was observed, but amplification of the non-target region that may be erroneously determined to be amplification of a non-hemolytic enterotoxin (nhe) gene was not observed.
It was thus confirmed that a non-hemolytic enterotoxin (nhe) gene of *Bacillus cereus* could be specifically amplified using the PCR primer set including a primer having the base sequence of SEQ ID NO: 1 and a primer having the base sequence of SEQ ID NO:2.

### Experiment 2

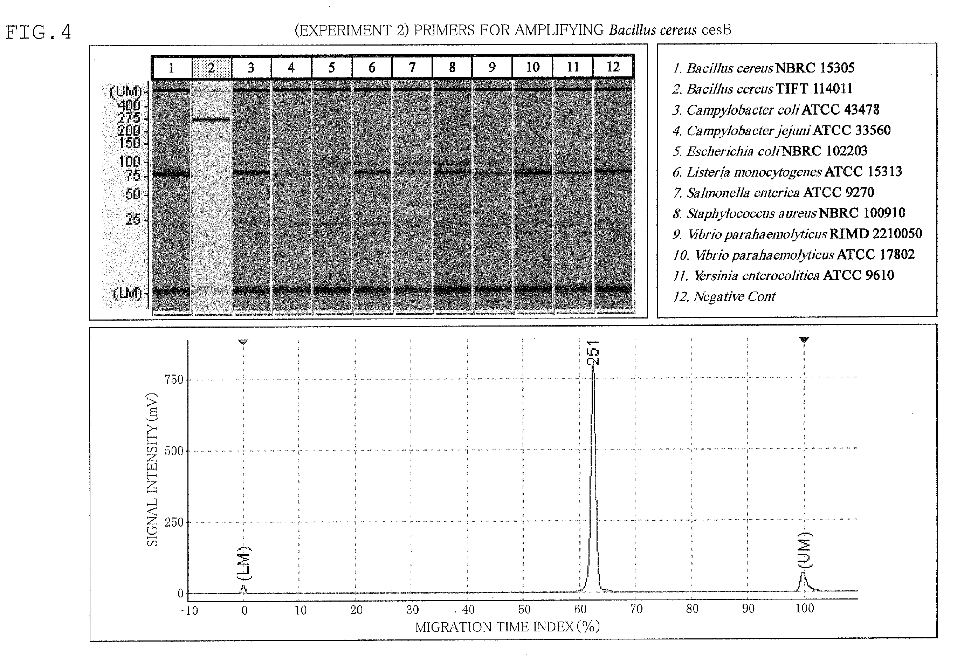

An experiment was performed in the same manner as in Experiment 1, except that a PCR primer set including a primer having the base sequence of SEQ ID NO: 3 and a primer having the base sequence of SEQ ID NO: 4 was used as the PCR primer set. The results are shown in FIG. 4.
The amplification target region of the PCR primer set used in Experiment 2 is a cereulide synthetase (cesB) gene in the toxin region of genomic DNA of *Bacillus cereus.* The length of the amplified product is 238 bp.

As shown in FIG. 4, a band was observed at around 250 bp when using genomic DNA of the strain 2 *(Bacillus cereus TIFT* 114011 having a cereulide synthetase (cesB) gene as the DNA sample, and no band was observed at around 250 bp when using the strains 1 and 3 to 12. The graph shown in FIG. 4 indicates the results obtained using the strain 2. As shown in FIG. 4, a peak was observed at 251 bp.

The above results suggest that the amplification target region was correctly amplified using the PCR primer set used in Experiment 2 when using the strain 2 (genomic DNA having a cereulide synthetase (cesB) gene) as the DNA sample.
When using the strains 1 and 3 to 11 (genomic DNA other than genomic DNA having a cereulide synthetase (cesB) gene) as the DNA sample, amplification of the non-target region that may be erroneously determined to be amplification of a cereulide synthetase (cesB) gene was not observed.
It was thus confirmed that a cereulide synthetase (cesB) gene of *Bacillus cereus* could be specifically amplified using the PCR primer set including a primer having the base sequence of SEQ ID NO: 3 and a primer having the base sequence of SEQ ID NO: 4.

### Experiment 3

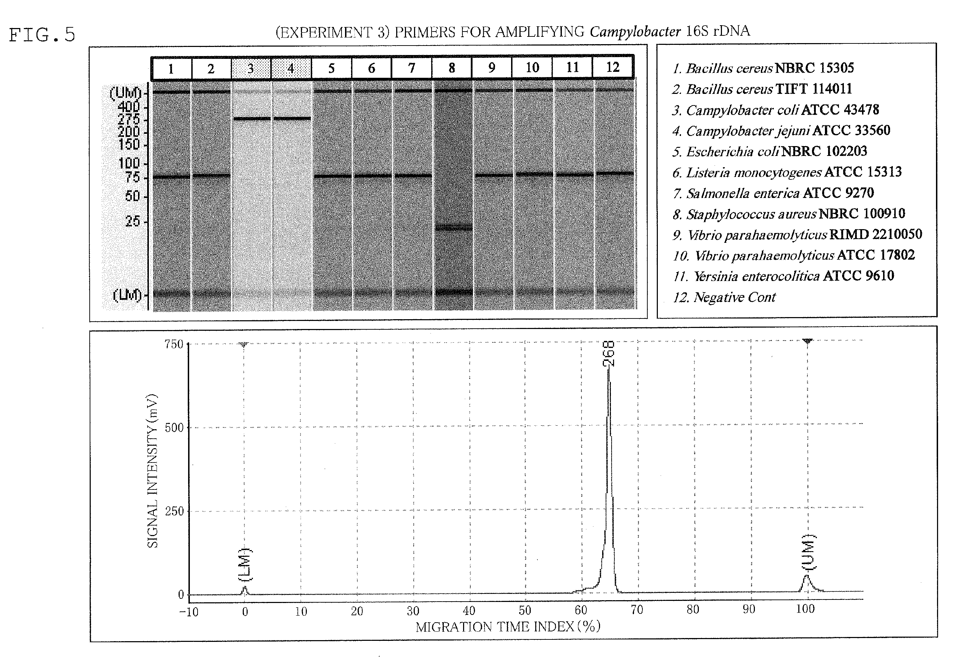

An experiment was performed in the same manner as in Experiment 1, except that a PCR primer set including a primer having the base sequence of SEQ ID NO: 5 and a primer having the base sequence of SEQ ID NO: 6 was used as the PCR primer set. The results are shown in FIG. 5.
The amplification target region of the PCR primer set used in Experiment 3 is a ribosomal (16S rDNA) gene in the essential region of genomic DNA of *Campylobacter.* The length of the amplified product is 263 bp.

As shown in FIG. 5, a band was observed at around 275 bp when using the strains 3 and 4 (genomic DNA having a ribosomal (16S rDNA) gene of *Campylobacter*) as the DNA sample, and no band was observed at around 275 bp when using the strains 1, 2, and 5 to 12. The graph shown in FIG. 5 indicates the results obtained using the strain 3. As shown in FIG. 5, a peak was observed at 268 bp.

The above results suggest that the amplification target region was correctly amplified using the PCR primer set used in Experiment 3 when using the strains 3 and 4 (genomic DNA having a ribosomal (16S rDNA) gene of *Campylobacter)* as the DNA sample.
When using the strains 1, 2, and 5 to 11 (genomic DNA other than genomic DNA having a ribosomal (16S rDNA) gene of *Campylobacter)* as the DNA sample, amplification of the non-target region that may be erroneously determined to be amplification of a ribosomal (16S rDNA) gene of *Campylobacterwas* not observed.
It was thus confirmed that a ribosomal (16S rDNA) gene of *Campylobacter* could be specifically amplified using the PCR primer set including a primer having the base sequence of SEQ ID NO: 5 and a primer having the base sequence of SEQ ID NO: 6.

### Experiment 4

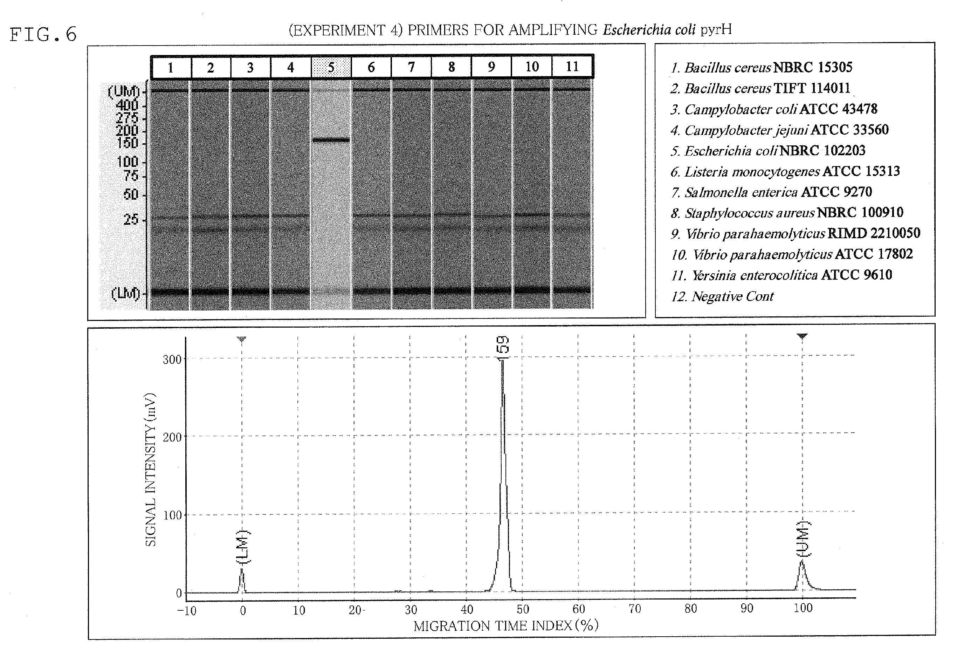

An experiment was performed in the same manner as in Experiment 1, except that a PCR primer set including a primer having the base sequence of SEQ ID NO: 7 and a primer having the base sequence of SEQ ID NO: 8 was used as the PCR primer set. The results are shown in FIG. 6.
The amplification target region of the PCR primer set used in Experiment 4 is a uridine monophosphate kinase (pyrH) gene in the essential region of genomic DNA of *Escherichia coli.* The length of the amplified product is 157 bp.

As shown in FIG. 6, a band was observed at around 160 bp when using the strain 5 (genomic DNA having a uridine monophosphate kinase (pyrH) gene of *Escherichia coli)* as the DNA sample, and no band was observed at around 160 bp when using the strains 1 to 4 and 6 to 12. The graph shown in FIG. 6 indicates the results obtained using the strain 5. As shown in FIG. 6, a peak was observed at 159 bp.

The above results suggest that the amplification target region was correctly amplified using the PCR primer set used in Experiment 4 when using the strain 5 (genomic DNA having a uridine monophosphate kinase (pyrH) gene of *Escherichia coli)* as the DNA sample.
When using the strains 1 to 4 and 6 to 11 (genomic DNA other than genomic DNA having a uridine monophosphate kinase (pyrH) gene of *Escherichia coli)* as the DNA sample, amplification of the non-target region that may be erroneously determined to be amplification of a uridine monophosphate kinase (pyrH) gene of *Escherichia coli* was not observed.
It was thus confirmed that a uridine monophosphate kinase (pyrH) gene of *Escherichia coli* could be specifically amplified using the PCR primer set including a primer having the base sequence of SEQ ID NO: 7 and a primer having the base sequence of SEQ ID NO: 8.

### Experiment 5

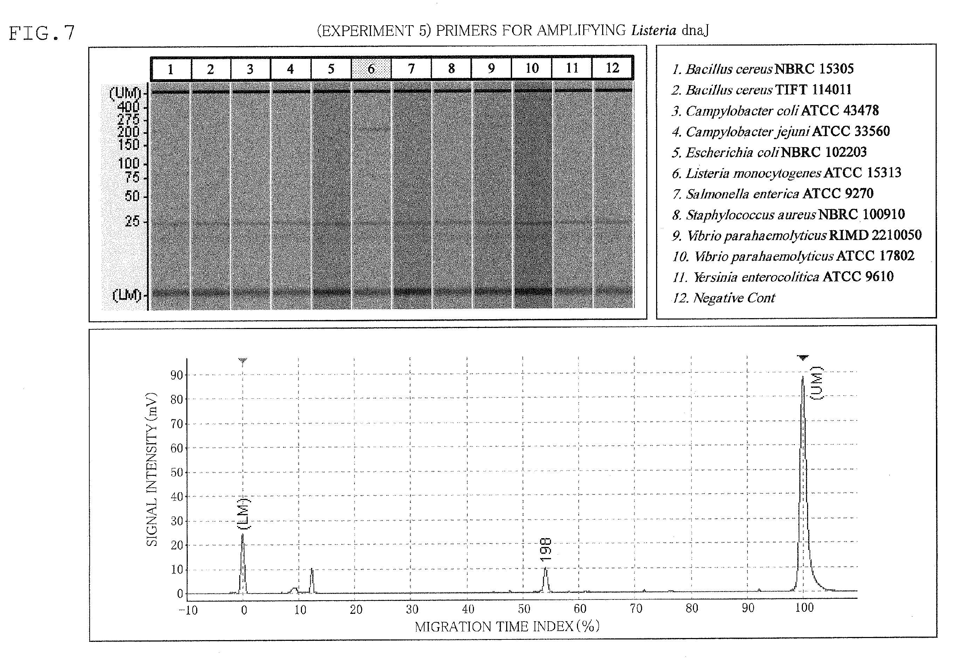

An experiment was performed in the same manner as in Experiment 1, except that a PCR primer set including a primer having the base sequence of SEQ ID NO: 9 and a primer having the base sequence of SEQ ID NO: 10 was used as the PCR primer set. The results are shown in FIG. 7.
The amplification target region of the PCR primer set used in Experiment 5 is a heat shock protein (dnaJ) gene in the essential region of genomic DNA of *Listeria.* The length of the amplified product is 176 bp.

As shown in FIG. 7, a band was observed at around 200 bp when using the strain 6 (genomic DNA having a heat shock protein (dnaJ) gene of *Listeria)* as the DNA sample, and no band was observed at around 200 bp when using the strains 1 to 5 and 7 to 12. The graph shown in FIG. 7 indicates the results obtained using the strain 6. As shown in FIG. 7, a peak was observed at 198 bp.

The above results suggest that the amplification target region was correctly amplified using the PCR primer set used in Experiment 5 when using the strain 6 (genomic DNA having a heat shock protein (dnaJ) gene of *Listeria)* as the DNA sample.
Note that the length of the amplified product obtained by Experiment 5 differs from the theoretical value by about 20 bp. It is considered that the above difference occurred due to insertion of a sequence into the heat-shock protein (dnaJ) gene of the strain 6.

When using the strains 1 to 5 and 7 to 11 (genomic DNA other than genomic DNA having a heat shock protein (dnaJ) gene of *Listeria)* as the DNA sample, amplification of the non-target region that may be erroneously determined to be amplification of a heat shock protein (dnaJ) gene of *Listeria* was not observed.
It was thus confirmed that a heat shock protein (dnaJ) gene of *Listeria* could be specifically amplified using the PCR primer set including a primer having the base sequence of SEQ ID NO: 9 and a primer having the base sequence of SEQ ID NO: 10.

### Experiment 6

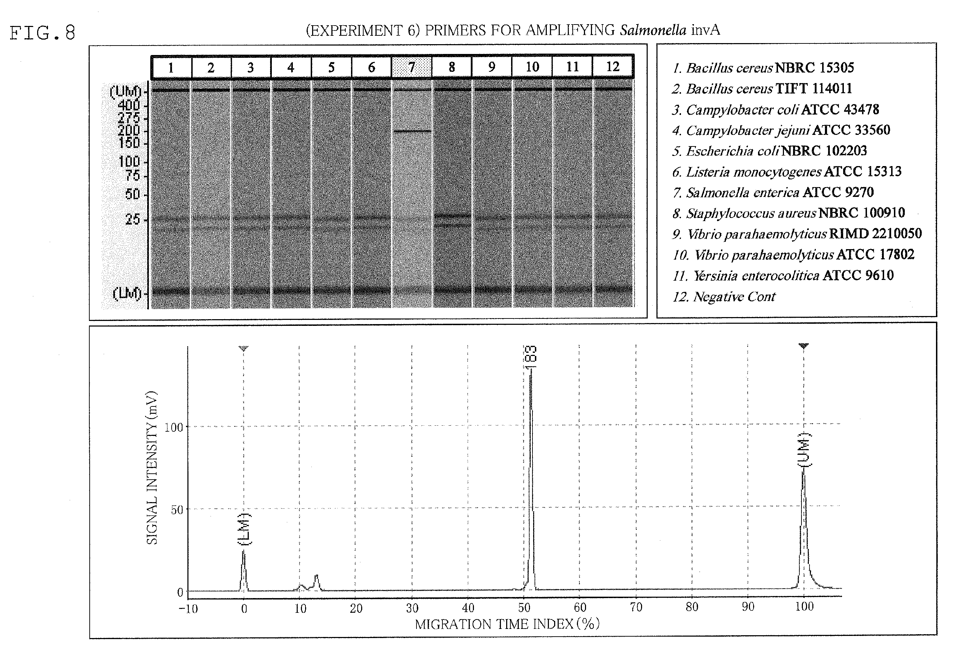

An experiment was performed in the same manner as in Experiment 1, except that a PCR primer set including a primer having the base sequence of SEQ ID NO: 11 and a primer having the base sequence of SEQ ID NO: 12 was used as the PCR primer set. The results are shown in FIG. 8.
The amplification target region of the PCR primer set used in Experiment 6 is an invasion (invA) gene in the toxin region of genomic DNA of *Salmonella.* The length of the amplified product is 180 bp.

As shown in FIG. 8, a band was observed at around 190 bp when using the strain 7 (genomic DNA having an invasion (invA) gene of *Salmonella)* as the DNA sample, and no band was observed at around 190 bp when using the strains 1 to 6 and 8 to 12. The graph shown in FIG. 8 indicates the results obtained using the strain 7. As shown in FIG. 8, a peak was observed at 183 bp.

The above results suggest that the amplification target region was correctly amplified using the PCR primer set used in Experiment 6 when using the strain 7 (genomic DNA having an invasion (invA) gene of *Salmonella)* as the DNA sample.
When using the strains 1 to 6 and 8 to 11 (genomic DNA other than genomic DNA having an invasion (invA) gene of *Salmonella)* as the DNA sample, amplification of the non-target region that may be erroneously determined to be amplification of an invasion (invA) gene of *Salmonella* was not observed.
It was thus confirmed that an invasion (invA) gene of *Salmonella* could be specifically amplified using the PCR primer set including a primer having the base sequence of SEQ ID NO: 11 and a primer having the base sequence of SEQ ID NO: 12.

### Experiment 7

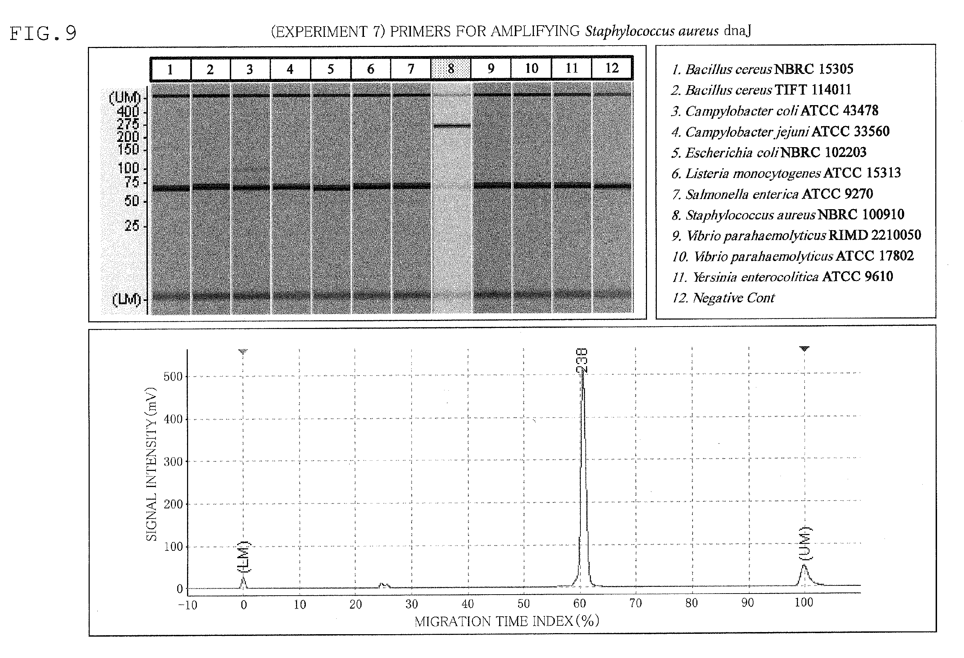

An experiment was performed in the same manner as in Experiment 1, except that a PCR primer set including a primer having the base sequence of SEQ ID NO: 13 and a primer having the base sequence of SEQ ID NO: 14 was used as the PCR primer set. The results are shown in FIG. 9.
The amplification target region of the PCR primer set used in Experiment 7 is a heat shock protein (dnaJ) gene in the essential region of genomic DNA of *Staphylococcus aureus.* The length of the amplified product is 236 bp.

As shown in FIG. 9, a band was observed at around 240 bp when using the strain 8 (genomic DNA having a heat shock protein (dnaJ) gene of *Staphylococcus aureus)* as the DNA sample, and no band was observed at around 240 bp when using the strains 1 to 7 and 9 to 12. The graph shown in FIG. 9 indicates the results obtained using the strain 8. As shown in FIG. 9, a peak was observed at 238 bp.

The above results suggest that the amplification target region was correctly amplified using the PCR primer set used in Experiment 7 when using the strain 8 (genomic DNA having a heat shock protein (dnaJ) gene of *Staphylococcus aureus)* as the DNA sample.
When using the strains 1 to 7 and 9 to 11 (genomic DNA other than genomic DNA having a heat shock protein (dnaJ) gene of *Staphylococcus aureus)* as the DNA sample, amplification of the non-target region that may be erroneously determined to be amplification of a heat shock protein (dnaJ) gene of *Staphylococcus aureus* was not observed.
It was thus confirmed that a heat shock protein (dnaJ) gene of *Staphylococcus aureus* could be specifically amplified using the PCR primer set including a primer having the base sequence of SEQ ID NO: 13 and a primer having the base sequence of SEQ ID NO: 14.

### Experiment 8

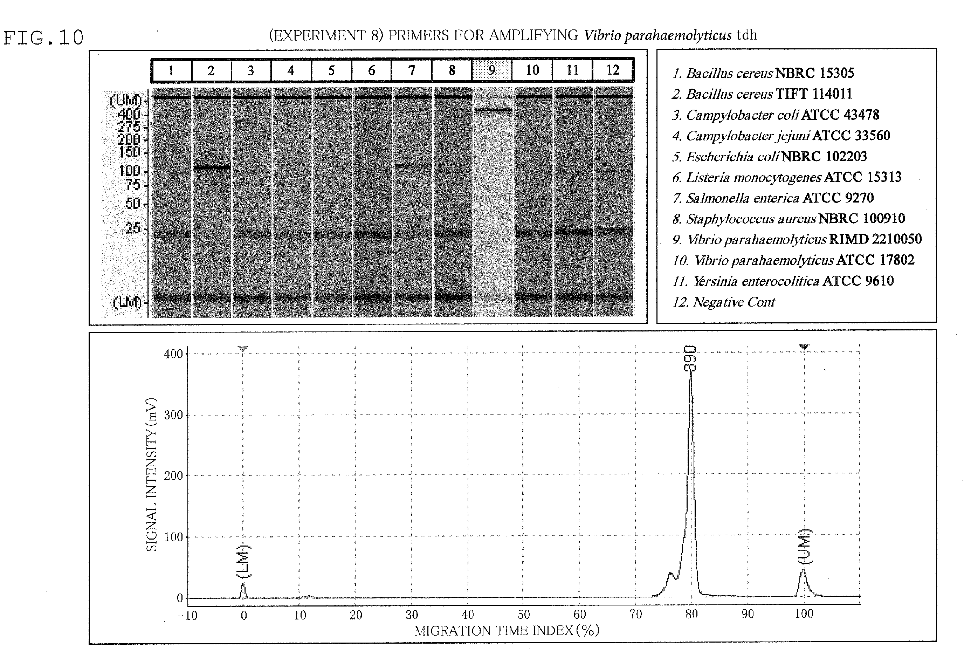

An experiment was performed in the same manner as in Experiment 1, except that a PCR primer set including a primer having the base sequence of SEQ ID NO: 15 and a primer having the base sequence of SEQ ID NO: 16 was used as the PCR primer set. The results are shown in FIG. 10.
The amplification target region of the PCR primer set used in Experiment 8 is a thermostable direct hemolysin (tdh) gene in the toxin region of genomic DNA of *Vibrio parahaemolyticus.* The length of the amplified product is 380 bp.

As shown in FIG. 10, a band was observed at around 400 bp when using the strain 9 (genomic DNA having a thermostable direct hemolysin (tdh) gene of *Vibrio parahaemolyticus)* as the DNA sample, and no band was observed at around 400 bp when using the strains 1 to 8 and 10 to 12. The graph shown in FIG. 10 indicates the results obtained using the strain 9. As shown in FIG. 10, a peak was observed at 390 bp. Note that the strain 10 *(Vibrio parahaemolyticus* ATCC 17802) is a *Vibrio parahaemolyticus* strain that does not have a thermostable direct hemolysin (tdh) gene, and amplification of the amplification target region was not observed when using the strain 10.

The above results suggest that the amplification target region was correctly amplified using the PCR primer set used in Experiment 8 when using the strain 9 (genomic DNA having a thermostable direct hemolysin (tdh) gene of *Vibrio parahaemolyticus)* as the DNA sample.
When using the strains 1 to 8, 10, and 11 (genomic DNA other than genomic DNA having a thermostable direct hemolysin (tdh) gene of *Vibrio parahaemolyticus)* as the DNA sample, amplification of the non-target region that may be erroneously determined to be amplification of a thermostable direct hemolysin (tdh) gene of *Vibrio parahaemolyticus* was not observed.
It was thus confirmed that a thermostable direct hemolysin (tdh) gene of *Vibrio parahaemolyticus* could be specifically amplified using the PCR primer set including a primer having the base sequence of SEQ ID NO: 15 and a primer having the base sequence of SEQ ID NO: 16.

It was thus confirmed that each PCR primer set according to the embodiment of the invention does not undergo a false-positive amplification reaction with genomic DNA of food poisoning bacteria other than the amplification target food poisoning bacteria.
In the following Examples 1 and 2, whether or not the target region can be specifically amplified when simultaneously using the above PCR primer sets was determined. The results are described below.

### Example 1

An experiment was performed using all of the PCR primer sets used in Experiments 1 to 8. Specifically, primers respectively having the base sequences of SEQ ID NO: 1 to 16 were added to a PCR reaction mixture used in Example 1.

The PCR reaction mixture used in Example 1 was prepared in the same manner as in Experiment 1 (except for the primers, the DNA sample, and sterilized water). The composition of the PCR reaction mixture is shown below.
Buffer solution "10×Ex Taq buffer" (20 mM Mg 2+ plus): 2.0 µl
Nucleic acid substrate "dNTP Mixture" (2.5 mM dATP, 2.5 mM dCTP, 2.5 mM dGTP, and 2.5 mM dTTP): 1.6 µl
primerF for detecting *Vibrio parahaemolyticus* (10 ng/µl, final conc.: 4 ng): 0.4 µl
primerR for detecting *Vibrio parahaemolyticus* (10 ng/µl, final conc.: 4 ng): 0.4 µl
Additional primerF (10 ng/µl, final conc.: 2 ng): 0.2 µl × 7
Additional primerR (10 ng/µl, final conc.: 2 ng): 0.2 µl × 7
Nucleic acid polymerase "EX Taq" (5 U/µl): 0.1 µl
DNA sample: 0.1 µl
Sterilized water: 11.7 µl

Genomic DNA of the following food poisoning bacteria strains 1 to 7 was used as the DNA sample, and separately subjected to PCR.
*1. Bacillus cereus* TIFT 114011
*2. Campylobacter jejuni* ATCC 33560
*3. Escherichia coli* NBRC 102203
*4. Listeria monocytogenes* ATCC 15313
*5. Salmonella enterica* ATCC 9270
*6. Staphylococcus aureus* NBRC 100910
*7. Vibrio parahaemolyticus* GTC 2055

PCR gene amplification was performed under the following conditions (the same conditions as those of Experiment 1) using a system "Mastercycler ep gradient" (manufactured by Eppendorf).
(1) 95°C/2min
(2) 95°C/10 sec (DNA strand separation step (denaturation step))
(3) 68°C/30 sec (annealing step)
(4) 72°C/30 sec (DNA synthesis step)
(5) 72°C/2 min
The steps (2) to (4) were repeated in 40 cycles.

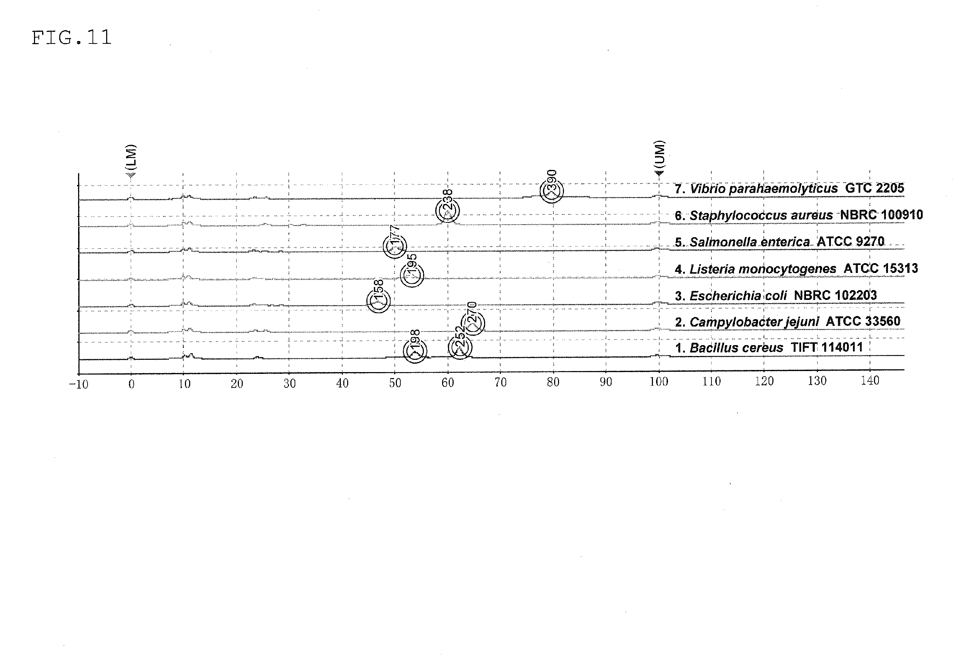

The PCR product was subjected to electrophoresis corresponding to each amplification target region to check whether or not a correct amplified product was obtained (i.e., whether or not the amplification target region was amplified). The results are shown in FIG. 11.

As shown in FIG. 11, the following peaks were observed for each PCR amplified product when using the strains 1 to 7 as the DNA sample.
*1. Bacillus cereus* TBFT 114011: 198 bp and 252 bp
*2. Campylobacter jejuni* ATCC 33560: 270 bp
*3. Escherichia coli* NBRC 102203: 158 bp
*4. Listeria monocytogenes* ATCC 15313: 195 bp
*5. Salmonella enterica* ATCC 9270: 177 bp
*6. Staphylococcus aureus* NBRC 100910: 238 bp
*7. Vibrio parahaemolyticus* GTC 2055: 390 bp

These results are approximate to the theoretical value. Therefore, it is considered that the amplification target region of the respective food poisoning bacteria could be specifically amplified using the PCR primer set according to the embodiment of the invention.
Note that the length (195 bp) of the amplified product of the strain 4 (*Listeria monocytogenes*) was longer than the length (176 bp) of the amplified product of *Listeria monocytogenes* shown in FIG. 1 by about 20 bp. It is considered that the above difference occurred due to insertion of a sequence.

### Example 2

Genomic DNA of all of the food poisoning bacteria strains 1 to 7 used in Example 1 was added to a PCR reaction mixture used in Example 2 as the DNA sample. The PCR reaction mixture used in Example 2 was prepared in the same manner as in Example 1 (except for the DNA sample and sterilized water). The composition of the PCR reaction mixture is shown below.
Buffer solution "10×Ex Taq buffer" (20 mM Mg 2+ plus): 2.0 µl
Nucleic acid substrate "dNTP Mixture" (2.5 mM dATP, 2.5 mM dCTP, 2.5 mM dGTP, and 2.5 mM dTTP): 1.6 µl
primerF for detecting *Vibrio parahaemolyticus* (10 ng/µl, final conc.: 4 ng): 0.4 µl
primerR for detecting *Vibrio parahaemolyticus* (10 ng/µl, final conc.: 4 ng): 0.4 µl
Additional primerF (10 ng/µl, final conc.: 2 ng): 0.2 µl × 7
Additional primerR (10 ng/µl, final conc.: 2 ng): 0.2 µl × 7
Nucleic acid polymerase "EX Taq" (5 U/µl): 0.1 µl
DNA sample: 0.1 µl × 7
Sterilized water: 5.7 µl

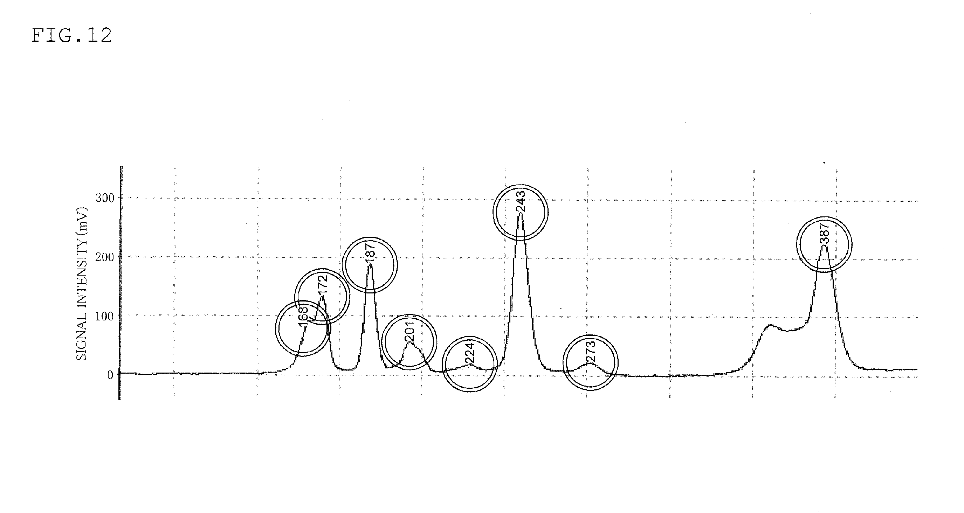

The amplification target region was simultaneously amplified by PCR under the same conditions as those of Example 1 using the above PCR reaction mixture.
The amplified product was subjected to electrophoresis using an electrophoresis system "MultiNA" (manufactured by Shimadzu Corporation). The results are shown in FIG. 12.

As shown in FIG. 12, the following eight peaks were observed.
(1) 168 bp, (2) 172 bp, (3) 187 bp, (4) 201 bp, (5) 224 bp, (6) 243 bp, (7) 273 bp, (8) 387 bp
It is considered that these peaks can be respectively attributed to the amplified products of the following strains taking account of the results obtained in Example 1.

(1) 3. *Escherichia coli* NBRC 102203
(2) *5. Salmonella enterica* ATCC 9270
(3) *4. Listeria monocytogenes* ATCC 15313
(4) *1. Bacillus cereus* TIFT 114011
(5) 6. *Staphylococcus aureus* NBRC 100910
(6) 1. *Bacillus cereus* TIFT 114011
(7) *2. Campylobacter jejuni* ATCC 33560
(8) *7. Vibrio parahaemolyticus* GTC 2055

It was thus confirmed that *Bacillus cereus, Campylobacter, Escherichia coli, Listeria, Salmonella, Staphylococcus aureus,* and *Vibrio parahaemolyticus* could be simultaneously and specifically amplified using the PCR primer sets according to the embodiment of the invention.
As a result, it was thus confirmed that these food poisoning bacteria can be simultaneously and specifically detected according to the embodiment of the invention.

The invention is not limited to the above embodiments and examples. Various modifications may be made without departing from the scope of the invention.
For example, the components of the PCR reaction mixture may be appropriately changed as long as the PCR reaction mixture includes the PCR primer set according to the embodiment of the invention. The primer design method according to the embodiment of the invention may also be applied when designing primers for amplifying genomic DNA of other bacteria.

### INDUSTRIAL APPLICABILITY

The invention may suitably be used when it is desired to simultaneously, specifically, and quickly determine (detect) whether or not *Bacillus cereus, Campylobacter, Escherichia coli, Listeria, Salmonella, Staphylococcus aureus,* and/or *Vibrio parahaemolyticus* are present in food, the production environment, and the like, for example.

### SEQUENCE LISTING

### TSK1120PCTSequenceFile

## Claims

1. A PCR primer set for amplifying nucleic acid by PCR, the PCR primer set comprising two or more primer sets selected from a group consisting of a first primer set for amplifying DNA of *Bacillus cereus,* the first primer set comprising a primer having a base sequence of SEQ ID NO: 1 and a primer having a base sequence of SEQ ID NO: 2, a second primer set for amplifying DNA of *Bacillus cereus,* the second primer set comprising a primer having a base sequence of SEQ ID NO: 3 and a primer having a base sequence of SEQ ID NO: 4, a third primer set for amplifying DNA of *Campylobacter,* the third primer set comprising a primer having a base sequence of SEQ ID NO: 5 and a primer having a base sequence of SEQ ID NO: 6, a fourth primer set for amplifying DNA of *Escherichia coli,* the fourth primer set comprising a primer having a base sequence of SEQ ID NO: 7 and a primer having a base sequence of SEQ ID NO: 8, a fifth primer set for amplifying DNA of *Listeria,* the fifth primer set comprising a primer having a base sequence of SEQ ID NO: 9 and a primer having a base sequence of SEQ ID NO: 10, a sixth primer set for amplifying DNA of *Salmonella,* the sixth primer set comprising a primer having a base sequence of SEQ ID NO: 11 and a primer having a base sequence of SEQ ID NO: 12, a seventh primer set for amplifying DNA of *Staphylococcus aureus,* the seventh primer set comprising a primer having a base sequence of SEQ ID NO: 13 and a primer having a base sequence of SEQ ID NO: 14, and an eighth primer set for amplifying DNA of *Vibrio parahaemolyticus,* the eighth primer set comprising a primer having a base sequence of SEQ ID NO: 15 and a primer having a base sequence of SEQ ID NO: 16.

2. The PCR primer set according to claim 1, the PCR primer set comprising all of the first primer set, the second primer set, the third primer set, the fourth primer set, the fifth primer set, the sixth primer set, the seventh primer set, and the eighth primer set.

3. The PCR primer set according to claim 1 or 2, wherein the primers included in the first primer set, the second primer set, the third primer set, the fourth primer set, the fifth primer set, the sixth primer set, the seventh primer set, and the eighth primer set are (A) a primer having a corresponding base sequence among the base sequences of SEQ ID NO: 1 to 16 wherein one or a plurality of bases are deleted, substituted, or added, or (B) a primer that comprises a nucleic acid fragment that hybridizes to a nucleic acid fragment having a base sequence complementary to a corresponding base sequence among the base sequences of SEQ ID NO: 1 to 16 under stringent conditions.

4. A PCR primer set comprising primers respectively having a base sequence complementary to those of the PCR primer set according to any one of claims 1 to 3.

5. A PCR primer set for amplifying nucleic acid by PCR, the PCR primer set comprising two or more primer sets selected from a group consisting of a primer set for amplifying a non-hemolytic enterotoxin (nhe) gene contained in genomic DNA of *Bacillus cereus,* a primer set for amplifying a cereulide synthetase (cesB) gene contained in genomic DNA of *Bacillus cereus,* a primer set for amplifying a ribosomal (16S rDNA) gene contained in genomic DNA of *Campylobacter,* a primer set for amplifying a uridine monophosphate kinase (pyrH) gene contained in genomic DNA of *Escherichia coli,* a primer set for amplifying a heat shock protein (dnaJ) gene contained in genomic DNA of *Listeria,* a primer set for amplifying an invasion (invA) gene contained in genomic DNA of *Salmonella,* a primer set for amplifying a heat shock protein (dnaJ) gene contained in genomic DNA of *Staphylococcus aureus,* and a primer set for amplifying a thermostable direct hemolysin (tdh) gene contained in genomic DNA of *Vibrio parahaemalyticus.*

6. A PCR reaction mixture comprising the PCR primer set according to any one of claims 1 to 5.

7. A method for detecting food poisoning bacteria comprising specifically amplifying nucleic acid contained in a PCR amplification target sample using two or more primer sets to obtain an amplified product, when the PCR amplification target sample contains nucleic acid of one or two or more types of food poisoning bacteria that correspond to the two or more primer sets, and detecting the one or two or more types of food poisoning bacteria based on the amplified product, the two or more primer sets being selected from a group consisting of a first primer set for amplifying DNA of *Bacillus cereus,* the first primer set comprising a primer having a base sequence of SEQ ID NO: 1 and a primer having a base sequence of SEQ ID NO: 2, a second primer set for amplifying DNA of *Bacillus cereus,* the second primer set comprising a primer having a base sequence of SEQ ID NO: 3 and a primer having a base sequence of SEQ ID NO: 4, a third primer set for amplifying DNA of *Campylobacter,* the third primer set comprising a primer having a base sequence of SEQ ID NO: 5 and a primer having a base sequence of SEQ ID NO: 6, a fourth primer set for amplifying DNA of *Escherichia coli,* the fourth primer set comprising a primer having a base sequence of SEQ ID NO: 7 and a primer having a base sequence of SEQ ID NO: 8, a fifth primer set for amplifying DNA of *Listeria,* the fifth primer set comprising a primer having a base sequence of SEQ ID NO: 9 and a primer having a base sequence of SEQ ID NO: 10, a sixth primer set for amplifying DNA of *Salmonella,* the sixth primer set comprising a primer having a base sequence of SEQ ID NO: 11 and a primer having a base sequence of SEQ ID NO: 12, a seventh primer set for amplifying DNA of *Staphylococcus aureus,* the seventh primer set comprising a primer having a base sequence of SEQ ID NO: 13 and a primer having a base sequence of SEQ ID NO: 14, and an eighth primer set for amplifying DNA of *Vibrio parahaemolyticus,* the eighth primer set comprising a primer having a base sequence of SEQ ID NO: 15 and a primer having a base sequence of SEQ ID NO: 16.

8. The method for detecting food poisoning bacteria according to claim 7, further comprising specifically amplifying nucleic acid contained in the PCR amplification target sample using all of the first primer set, the second primer set, the third primer set, the fourth primer set, the fifth primer set, the sixth primer set, the seventh primer set, and the eighth primer set, when the PCR amplification target sample contains nucleic acid of one or two or more types of food poisoning bacteria that correspond to the first primer set, the second primer set, the third primer set, the fourth primer set, the fifth primer set, the sixth primer set, the seventh primer set, and/or the eighth primer set.

9. The method for detecting food poisoning bacteria according to claim 7 or 8, wherein the primers included in the first primer set, the second primer set, the third primer set, the fourth primer set, the fifth primer set, the sixth primer set, the seventh primer set, and the eighth primer set are (A) a primer having a corresponding base sequence among the base sequences of SEQ ID NO: 1 to 16 wherein one or a plurality of bases are deleted, substituted, or added, or (B) a primer that comprises a nucleic acid fragment that hybridizes to a nucleic acid fragment having a base sequence complementary to a corresponding base sequence among the base sequences of SEQ ID NO: 1 to 16 under stringent conditions.

10. A method for detecting food poisoning bacteria comprising specifically amplifying nucleic acid contained in a PCR amplification target sample using primer sets that comprise primers respectively having a base sequence complementary to those of the primer sets used in the method for detecting food poisoning bacteria according to any one of claims 7 to 9, when the PCR amplification target sample contains nucleic acid of one or two or more types of food poisoning bacteria that correspond to the primer sets.

11. The method for detecting food poisoning bacteria according to any one of claims 7 to 10, further comprising subjecting the amplified product to electrophoresis to simultaneously detect the one or two or more types of food poisoning bacteria.
